# EUROPEAN PATENT APPLICATION

(11) **EP 1 806 133 A1**
(43) Date of publication of application: **11.07.2007**
(21) Application number: 05793078.6
(22) Date of filing: 14.10.2005
(51) Int. Cl.: A61K 31/197, A61K 9/72, A61P 3/06, A61P 3/10, A61P 9/12, A61P 25/22, A61P 25/28

(54) **METHOD FOR MODIFYING THE AMBIENCE, AND SPRAY LIQUID AND SPRAYER USED IN THE METHOD**

(30) Priority: 14.10.2004 JP 2004299687
(71) Applicant: DAIKIN INDUSTRIES, LTD., Kita-ku, Osaka 530-8323 (JP)
(72) Inventor: ARAI, Jun-ichiro, DAIKIN INDUSTRIES, LTD., Kusatsu-shi Shiga 525-8526 (JP); YAMASHITA, Mitsugu, Daikin Industries, Ltd., Kusatsu-shi Shiga 525-8526 (JP)
(74) Representative: Goddar, Heinz J.
(86) International application number: PCT/JP2005/018957
(87) International publication number: WO 2006/041158

(57) **Abstract**

A solution including a solute of γ-aminobutyric acid dissolved in a solvent is sprayed in a space.

## Description

### Technical Field

The present invention relates to an atmosphere modifying method for a body space, a room space or the like, and a spray agent and a spray device used in the method.

### Background Art

It has been confirmed that γ-aminobutyric acid (hereinafter referred to as the "GABA") has a variety of pharmacological actions. The pharmacological actions are, for example, a blood pressure adjusting action for attaining a normal blood pressure, an action for suppressing increase of cholesterol and neutral fat in blood, an action for activating the operation of a kidney, a liver or a pancreas, an action for suppressing increase of a blood glucose level, an action for activating the metabolism of brain cells by attaining a good blood flow to the brain, an action for preventing fatness, an action for accelerating the metabolism of alcohol, an action for deodorizing body odor or bad breath, an action for dissolving emotional disorder or anxiety neurosis, an action for improving the aftereffect of apoplexy, an action for suppressing colon cancer, and an action for accelerating the discharge of growth hormone. Therefore, attention has recently been earnestly paid to the GABA as a supplement.

Patent Document 1 discloses that ingestion of the GABA directly increases appearance of alpha waves so as to reduce appearance of beta waves.

Patent Document 2 discloses a method for fabricating a GABA-containing diet in which a microorganism having protease productivity and a microorganism having glutamate decarboxylase productivity are seeded and cultured in a food material including protein or peptide having glutamic acid as constitutive amino acid. According to the description of this document, a GABA-containing diet having a good taste and a good color tone can be efficiently obtained without using glutamic acid, that is, a food additive.

When the GABA is ingested, however, it cannot reach the brain because it cannot pass through a cerebral blood vessel barrier, and it is disadvantageously absorbed in the small intestine and then metabolized in passing through the liver. Therefore, a GABA analogue free from such a problem is used instead of the GABA itself.

Patent Document 3 discloses a medicine composition including an effective amount of GABA analogue. According to the description of this document, insomnia of a mammal can be cured by this composition.

Each of Patent Documents 4 and 5 discloses oral administration or parenteral administration such as nasal administration of a GABA analogue. However, these documents do not mention any specific means for the nasal administration.

An object of the invention is providing an atmosphere modifying method in which the GABA can be taken into a body by a method different from a conventional method, and a spray agent and a spray device for use in the atmosphere modifying method.
Patent Document 1: Japanese Laid-Open Patent Publication No. 2003-252755
Patent Document 2: Japanese Laid-Open Patent Publication No. 2000-14356
Patent Document 3: Japanese Laid-Open Patent Publication No. 2002-520277
Patent Document 4: Japanese Laid-Open Patent Publication No. 2001-131161
Patent Document 5: Japanese Laid-Open Patent Publication No. 2001-342150

### Disclosure of the Invention

In the atmosphere modifying method of this invention, a solution including a solute of GABA dissolved in a solvent is sprayed in a space.

In this manner, since a GABA solution is sprayed in a space, the GABA is absorbed through nasal mucosa or a lung, and hence, the GABA can be taken into a body without taking a pill or the like.

Also, the GABA is absorbed in a paranasal cavity through nasal mucosa so as to enter not only blood vessels but also lymph, and the GABA having entered the lymph does not pass through a cerebral blood vessel barrier, and hence, not an analogue of the GABA but the GABA itself can be taken into a brain.

Furthermore, since the GABA is absorbed through nasal mucosa or a lung and does not pass through a portal vein, metabolism caused through the first pass of a liver can be avoided.

Moreover, in the case where the GABA solution is sprayed in a room space while a person is sleeping therein, the concentration of the GABA in blood can be kept high for a long period of time, and hence, the discharge of the growth hormone can be accelerated.

In the atmosphere modifying method of this invention, the solution is sprayed in a particle diameter of preferably 10 µm or less and more preferably 5 µm or less.

In this manner, since the particle diameter of the sprayed solution is sufficiently small, the solution can easily enter a nose or a lung and can be smoothly absorbed. When the particle diameter of the sprayed solution is larger than 10 µm, a large amount of the solution does not enter the nose or the lung but enters a stomach through a throat. Also, in order to keep a constant concentration in the space, particles released in the space preferably have a particle diameter not falling down in a short period of time. When the particle diameter is 10 µm, the particles fall down by 60 cm per minute, and the falling distance is in proportion to a square of the particle diameter, when the particle diameter is 1 µm, the particles fall down by 0.6 cm per minute, and when the particle diameter is 5 µm, the particles fall down by 15 through 20 cm per minute. In consideration of convection, the particle diameter is preferably 5 µm or less. It is noted that the particle diameter of the sprayed solution herein means an average particle diameter.

In the atmosphere modifying method of this invention, a concentration of the GABA of 10 µg/m³ through 100 mg/m³ is preferably attained in the space by the spray.

Thus, an appropriate amount of GABA can be taken into a body. When the concentration of the GABA in the space is lower than 10 µg/m³, the effects attained by taking the GABA into a body are reduced. On the other hand, when the concentration of the GABA in the space is higher than 100 mg/m³, it cannot be expected that the effects are more remarkably attained.

In the atmosphere modifying method of the invention, the spray of the solution including the GABA may be controlled in such a manner that a spray amount per unit time is larger at the start of the spray than in a steady spray time.

Thus, the concentration of the GABA in the space can be increased in a short period of time after starting the spray.

In the atmosphere modifying method of the invention, the spray of the solution including the GABA may be controlled in such a manner that a concentration of the GABA in the space is constant.

Thus, the GABA can be stably and continuously taken into a body.

In the atmosphere modifying method of this invention, the spray of the solution including the GABA may be controlled to start when a given period of time elapses after detecting start of sleep.

Thus, since the efficiency for taking the GABA is the highest when a given period of time elapses after starting sleeping, the discharge of the growth hormone can be thus effectively accelerated.

The spray agent used in the atmosphere modifying method of this invention is to be sprayed in a space and includes a solute of GABA dissolved in a solvent.

In the spray agent of this invention, a concentration of the GABA is preferably 0.0097 through 3.88 mol/1.

In the aforementioned manner, an appropriate amount of the GABA can be taken into a body and the liquid properties of the spray agent can be appropriate. Furthermore, the spray agent is applicable to both a case where the spray amount per unit time is large with the spray agent replenished in a short cycle (of, for example, 1 day) and a case where the spray amount per unit time is small with the spray agent replenished in a long cycle (of, for example, two or three months). When the concentration of the GABA is lower than 0.0097 mol/1, it is necessary to increase the particle diameter and the spray amount for attaining the effects, and hence, the concentration in the space is not stable owing to the influence of fall and the like, and when the particle diameter is reduced for suppressing the fall, the amount to be taken into a body is reduced, and hence, the effects cannot be sufficiently attained. On the other hand, when the concentration of the GABA is higher than 3.88 mol/1, the viscosity is so high that the spray agent is difficult to spray, or there may arise a problem that the GABA is deposited. Accordingly, in the spray agent of this invention, the concentration of the GABA is more preferably 1.94 mol/1 or less. In particular, such a concentration is suitably employed in the case where the spray amount per unit time is small with the spray agent replenished in a short cycle. It is noted that a concentration of the GABA of 0.0097 through 3.88 mol/1 corresponds to 0.1 through 40% by mass when the solvent is water.

The spray agent of this invention can be sprayed in a space by using a spray device such as a nebulizer.

### Brief Description of Drawings

[FIG. 1] FIG. **1** is a graph for showing the relationship between particle diameters of a solution and their ratios obtained in spray by a nebulizer of a supersonic system.
[FIG. 2] FIG. **2** is a graph for showing the relationship between particle diameters of a solution and their ratios obtained in spray by a spray device of an ink jet system.
[FIG. 3] FIG. **3** is a graph for showing the relationship between particle diameters of a solution and their ratios obtained in spray by a spray device of an electrostatic atomizing system.
[FIG. 4] FIG. **4** is a schematic diagram for showing the architecture of an electrostatic spray device (10).
[FIG. 5] FIG. **5** is a perspective view of a spray cartridge (15).
[FIG. 6] FIG. **6** is a cross-sectional view of a principal part of the spray cartridge (15).
[FIG. 7] FIG. **7A** is a cross-sectional view of a tip of a spray nozzle (31) obtained during spray and FIG. **7B** is a cross-sectional view of the tip of the spray nozzle (31) obtained in stopping the spray.

### Best Mode for Carrying Out the Invention

Now, a preferred embodiment of an atmosphere modifying method for a space will be described in detail.

In the atmosphere modifying method for a space of this embodiment, a spray agent including GABA is sprayed in a space with a spray device, so that the GABA can be taken into a human body by absorption through nasal mucosa or a lung.

The spray agent is basically a solution in which the GABA is dissolved as a solute in a solvent. The spray agent includes, in addition, an electric conductivity adjustor, a dissolution assisting agent, a suspending agent, an isotonic agent, a buffer, an indolent agent or the like, and may include an additive such as an antiseptic, an antioxidant, a colorant, a sweetner or a perfume if necessary.

The GABA is represented by a formula below. The GABA is an inhibitory neuron-transmitter present in a high concentration in a central nervous system of a mammal, and can be artificially synthesized. Furthermore, the GABA exhibits pharmacological actions such as a blood pressure adjusting action for attaining a normal blood pressure, an action for suppressing increase of cholesterol and neutral fat in blood, an action for activating the operation of a kidney, a liver or a pancreas, an action for suppressing increase of a blood glucose level, an action for activating the metabolism of brain cells by attaining a good blood flow to the brain, an action for preventing fatness, an action for accelerating the metabolism of alcohol, an action for deodorizing body odor or bad breath, an action for dissolving emotional disorder or anxiety neurosis, an action for improving the aftereffect of apoplexy, an action for suppressing colon cancer, and an action for accelerating the discharge of growth hormone.

H₂NCH₂CH₂CH₂COOH

Examples of the solvent are water (including injectable water, normal saline and a Ringer solution), alcohol, propylene glycol, polyethylene glycol, sesame oil, corn oil, olive oil and cotton seed oil.

Examples of the electric conductivity adjustor are ethanol and isopropanol.

Examples of the dissolution assisting agent are polyethylene glycol, propylene glycol, D-mannitol, trehalose, benzyl benzoate, ethanol, isopropanol, tris-amino methane, cholesterol, triethanolamine, sodium carbonate, sodium citrate, sodium salicylate and sodium acetate.

Examples of the suspending agent are stearyl triethanolamine, sodium lauryl sulfate, lauryl aminopropionic acid, lecithin, benzalkonium chloride, benzethonium chloride, a surface active agent (such as glycerin monostearate), a hydrophilic polymer (such as polyvinyl alcohol, poly(vinylpyrrolidone), sodium carboxymethyl cellulose, methyl cellulose, hydroxymethyl cellulose, hydroxyethyl cellulose and hydroxypropyl cellulose), Polysorbate, and polyoxyethylene-hardened castor oil.

Examples of the isotonic agent are sodium chloride, glycerin, D-mannitol, D-sorbitol and glucose.

An example of the buffer is a buffer solution including phosphate, acetate, carbonate or citrate.

An example of the indolent agent is benzyl alcohol.

Examples of the antiseptic are parahydroxybenzoic acid esters, chlorobutanol, benzyl alcohol, phenetyl alcohol, dehydroacetic acid and sorbic acid.

Examples of the antioxidant are sulfite and ascorbate.

Examples of the colorant are water-soluble coloring tar dyes such as food dyes including food red No. 2 or 3, food yellow No. 4 or 5 and food blue No. 1 or 2, insoluble lake dyes such as aluminum salts of these water-soluble food tar dyes, and natural dyes such as β-carotene, chlorophyll and blood red.

Examples of the sweetner are sodium saccharin, glycyrrhizic acid dipotassium, aspartame and stevia.

An example of the perfume is a water-soluble essence with a flavor of lemon, lavender, Japanese cypress or the like.

In the spray agent, the concentration of the GABA is preferably 0.0097 through 3.88 mol/l and more preferably 0.0097 through 1.94 mol/l. When the concentration of the GABA falls in this range, an appropriate amount of GABA can be taken into a body and the liquid properties of the spray agent such as the viscosity and the solubility can be appropriate. Furthermore, the spray agent is applicable to both a case where the spray amount per unit time is large with the spray agent replenished in a short cycle (of, for example, 1 day) and a case where the spray amount per unit time is small with the spray agent replenished in a long cycle (of, for example, two or three months). In particular, the concentration of the GABA of 0.0097 through 1.94 mol/1 is suitably employed in the case where the spray amount per unit time is large with the spray agent replenished in a short cycle. It is noted that in a spray agent including the GABA dissolved in the solvent of water, the concentration of the GABA of 0.0097 through 3.88 mol/l corresponds to 0.1 through 40% by mass and the concentration of the GABA of 0.0097 through 1.94 mol/l corresponds to 0.1 through 20% by mass.

The spray agent has conductivity of preferably 50 through 1000 µs/cm and more preferably 100 through 300 µs/cm. When the conductivity falls in this range, the state of spraying the spray agent is so stable that no large particles are formed, and hence, adhesion of particles onto the tip of a tubular spray nozzle, that is, one factor of clogging, can be avoided. In order to reduce the conductivity, it is necessary to increase the proportion of petroleum in the agent, and the upper limit of the proportion is restricted by the Fire Services Act, and therefore, the aforementioned range is more preferable for obeying the law.

This spray agent has a viscosity of preferably 0.1 through 5.1 cP and more preferably 0.1 through 3.0 cP. When the viscosity falls in this range, a problem such as clogging is minimally caused in the spray device. In the case where the spray agent is sprayed through a tubular spray nozzle of 0.4 through 0.6 mm, even when the viscosity is high, it can be sprayed by, for example, increasing the applied voltage or increasing the conductivity, which is not preferable because ozone may be generated or the proportion of ethanol or the like in the solution should be increased.

The spray device is, for example, a nebulizer for use in inhale of a drug solution for spraying the spray agent in a body space of a human or an atomizer provided in an air conditioner for spraying the spray agent in a room space. A spraying system employed by the spray device is, for example, a ultrasonic system, a bubble-jet system, a piezoelectric system, an electrostatic atomizing system or the like.

In spraying the spray agent in a space by using the spray device, the spray agent can easily enter a nose or a lung to be smoothly absorbed when the particle diameter of the sprayed solution is sufficiently small, and therefore, the solution is sprayed in a particle diameter of preferably 10 µm or less and more preferably 5 µm or less. Furthermore, in order to take an appropriate amount of GABA into a body, the concentration of the GABA in the space attained by spraying the spray agent is 10 µg/m³ through 100 mg/m³. It is noted that the maximum intake of the GABA into a body is approximately 1000 mg per day, and in the case where the spray agent is sprayed in a room space, the spray agent is sprayed per day in an amount corresponding to approximately 10 g of the GABA.

In the atmosphere modifying method for a space described above, since the GABA solution is sprayed in a room, the GABA is taken by absorption through nasal mucosa or a lung, and hence, the GABA can be taken into a body without taking a pill or the like.

Also, the GABA is absorbed in a paranasal cavity through nasal mucosa so as to enter not only blood vessels but also lymph, and the GABA having entered the lymph does not pass through a cerebral blood vessel barrier, and hence, not an analogue of the GABA but the GABA itself can be taken into a brain.

Furthermore, since the GABA is absorbed through nasal mucosa or a lung and does not pass through a portal vein, metabolism caused through the first pass of a liver can be avoided.

Moreover, in the case where the GABA solution is sprayed in a room space while a person is sleeping therein, the concentration of the GABA in blood can be kept high for a long period of time, and hence, the discharge of the growth hormone can be accelerated.

Next, the architecture of an electrostatic spray device used for spraying the spray agent including the GABA will be described.

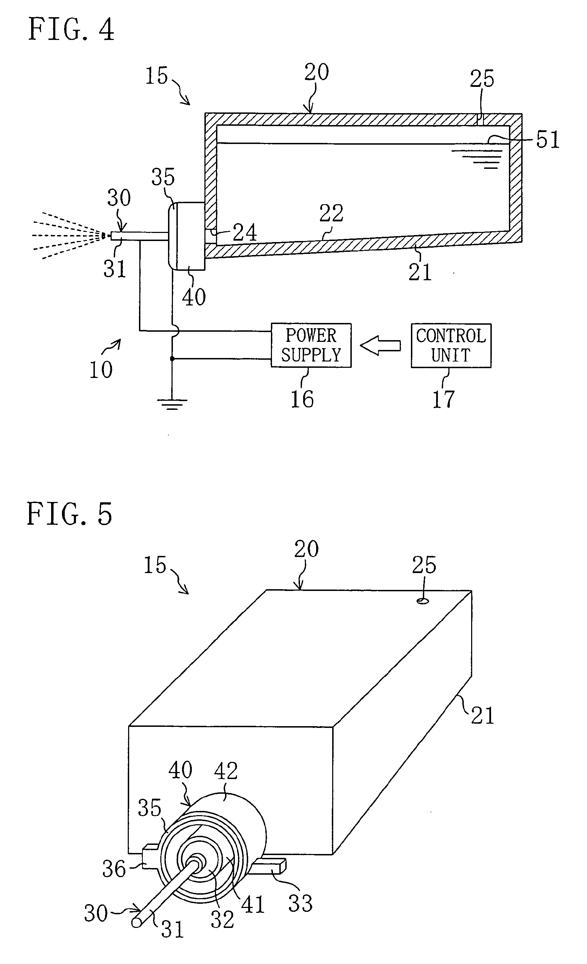

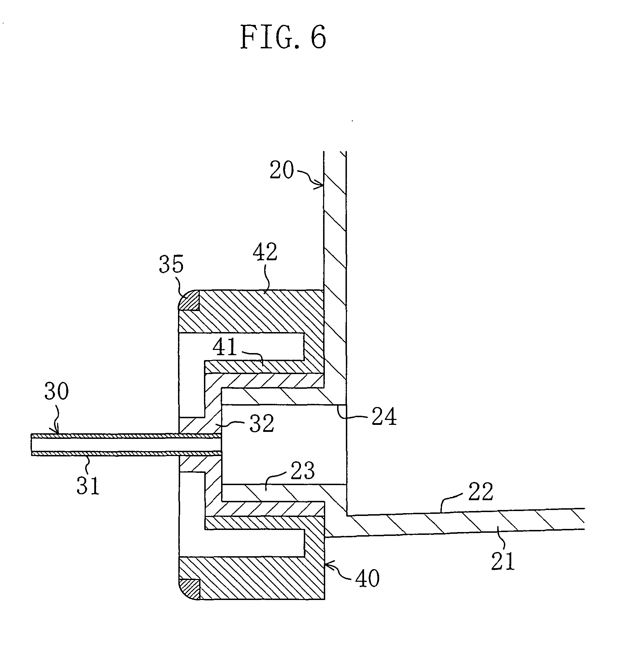

FIGS. 4 through 6 show an electrostatic spray device (10) according to the embodiment of the invention.

This electrostatic spray device (10) includes a spray cartridge (15), a power supply (16) and a control unit (17).

The spray cartridge (15) includes a solution tank (20), a nozzle unit (30), an electrode holder (40) and a ring electrode (35).

The solution tank (20) is equipped with a hollow tank body (21) having an air vent port (25) on a top thereof. A tubular portion (23) projecting in a horizontal direction is provided in a lower portion on the front face of the tank body (21). The tubular portion (23) is communicated with the tank body (21) through a through hole (24) formed on the front face of the tank body (21).

The nozzle unit (30) includes a tubular spray nozzle (31) with an inner diameter of, for example, 0.4 through 0.6 mm and a nozzle holder (32) in a shape of a cylindrical closed-end cap. The nozzle holder (32) is provided so as to cover the tubular portion (23). The base of the spray nozzle (31) is inserted into the center of the end of the nozzle holder (32), so that the spray nozzle (31) can be communicated with the tank body (21) through the tubular portion (23) and the through hole (24). The nozzle holder (32) is provided with a terminal portion (33) extending sideward from the periphery thereof. The nozzle holder (32) and the terminal portion (33) are constructed as an electrode made of a conducting resin, and the spray nozzle (31) is electrically connected to the electrode.

The electrode holder (40) includes an inner cylinder (41) and an outer cylinder (42) concentric with each other having a space therebetween and connected to each other at their bases. The electrode holder (40) is provided with the inner cylinder (41) fit on the nozzle holder (32). In the electrode holder (40), the ring electrode formed in a ring shape and having a terminal portion (36) made of a tongue and extending sideward is externally fit on the periphery of the tip of the outer cylinder (42).

The power supply (16) is a DC high voltage power supply. The power supply (16) is electrically connected to the spray nozzle (31) through the terminal portion (33) of the nozzle holder (32) at its positive terminal and to the terminal portion (36) of the ring electrode (35) at its grounded negative terminal.

The control unit (17) is constructed for controlling on/off switching of the power supply (16).

When the ambient temperature is reduced, it is apprehended that the viscosity of the spray agent stored in the tank body (21) is increased so as to clog the spray nozzle (31). Therefore, the tank body (21) may be provided with a heat insulating mechanism such as a heat insulating material or with a heating mechanism such as a panel heater.

Next, the operation for spraying the spray agent performed by the electrostatic spray device (10) will be described.

In this electrostatic spray device (10), the spray agent including the GABA is stored in the tank body (21) of the solution tank (20). In the spray agent, the concentration of the GABA is preferably 0.0097 through 3.88 mol/1 and more preferably 0.0097 through 1.94 mol/1, and the viscosity is preferably 0.1 through 5.1 cP and more preferably 0.1 through 3.0 cP. The conductivity is preferably 50 through 1000 µs/cm and more preferably 100 through 300 µs/cm. The position of a liquid level (51) within the tank body (21) is higher than the tip of the spray nozzle (31), and since there is such a head difference between the liquid level (51) within the tank body (21) and the tip of the spray nozzle (31), the spray agent contained in the tank body (21) is supplied to the tip of the spray nozzle (31).

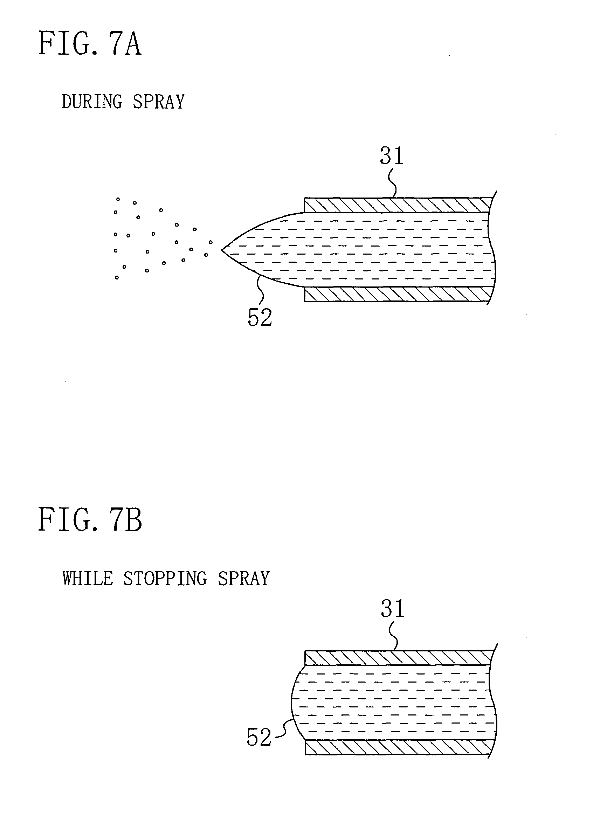

When the power supply (16) is turned on, a potential difference of, for example, approximately 3 through 7 kV is applied between the spray nozzle (31) and the ring electrode (35), so as to form an electric field in the vicinity of the tip of the spray nozzle (31). Also, the spray agent contained in the spray nozzle (31) is polarized, and + (plus) charges are collected in the vicinity of a gas-liquid interface (52) at the tip of the spray nozzle (31). Then, at the tip of the spray nozzle (31), the gas-liquid interface (52) is drawn into a cone shape as shown in FIG. 7A, and a part of the spray agent is changed into droplets as if the spray agent were pulled off from the apex of the cone-shaped gas-liquid interface (52), so as to be supplied in a room space. A person staying in the room inhales the droplets of the spray agent together with the air when he/she breathes. In order to allow the droplets included in the inspired air to reach air cells of the person, the particle diameter of the droplets is preferably as small as 10 µm or less and more preferably 5 µm or less.

When the power supply (16) is turned off, the spray nozzle (31) and the ring electrode (35) attain the same potential, and in the gas-liquid interface (52) formed at the tip of the spray nozzle (31), a state where the surface tension and the liquid pressure derived from the head difference are balanced as shown in FIG. 7B, and therefore, the aqueous solution never flows out from the tip of the spray nozzle (31). Specifically, even when a liquid pressure of, for example, 20 mm H₂O is applied to the gas-liquid interface (52) at the tip of the spray nozzle (31), the leakage of the aqueous solution (50) from the tip of the spray nozzle (31) can be prevented.

Next, operation control performed by the control unit (17) of the electrostatic spray device (10) will be described.

In this electrostatic spray device (10), the control unit (17) controls the spray operation for the spray agent by controlling a duty ratio, that is, a ratio between time when the power supply (16) is in an on state (on time) and time when the power supply (16) is in an off state (off time).

Specifically, in this electrostatic spray device (10), the duty ratio is set to be higher at the start of spray than in a steady spray time, so that the spray amount per unit time can be larger at the start of the spray than in the steady spray time. Thus, the concentration of the GABA in the space can be increased in a short period of time from the start of the spray.

Furthermore, in the steady spray time of this electrostatic spray device (10), the pressure to be applied to the spray agent by the spray nozzle (31) is changed in accordance with the height of the liquid level (51) within the solution tank (20). Therefore, the duty ratio is set in accordance with the height of the liquid level (51) (so that the duty ratio can be lower as the liquid level (51) is higher), and thus, the concentration of the GABA in the space can be controlled to be constant. In this manner, the GABA can be stably and continuously taken into a body.

Next, control of an operation for stirring the spray agent performed by the control unit (17) will be described.

At the tip of the spray nozzle (31), if the solvent is scattered from the spray agent, the solvent selectively attracts the electric field or concentration gradient is caused due to a difference in the kinetic momentum between the GABA and the solvent, and the viscosity of the spray agent is increased, which can be a factor of clogging the spray nozzle (31). This electrostatic spray device (10) is controlled so that, either during the spray of the spray agent or while stopping the spray, the power supply (16) can be repeatedly turned on/off (with a repeating frequency of, for example, 0.1 through 10.0 Hz) for a given period of time (of, for example, 5 through 20 seconds) at a given interval (of, for example, 1 through 10 minutes), and thus, the electric field is repeatedly formed and erased. When the electric field is formed by turning on the power supply (16), the spray agent is gradually swollen from the tip of the spray nozzle (31), and when the electric field is erased by turning off the power supply (16), the spray agent swollen from the tip of the spray nozzle (31) is withdrawn into the spray nozzle (31). When such formation and erasure of the electric field are repeated, the spray agent repeatedly goes out of and withdraws into the spray nozzle (31), and is stirred through this movement. As a result, the concentration increase of the spray agent at the tip of the spray nozzle (31) can be suppressed.

Next, control for an in-sleep operation mode performed by the control unit (17) will be described.

In this electrostatic spray device (10), when the in-sleep operation mode is selected, start of sleep of a person is detected by a sleep detecting sensor (not shown) connected to the control unit (17), so that the spray of the spray agent can be controlled to start when a given period of time (of, for example, 1 hour) elapses after the detection of the sleep. Since the efficiency for taking the GABA is the highest when a given period of time elapses after starting sleeping, the discharge of the growth hormone can be thus effectively accelerated.

In addition, an appropriate spray amount may be set by inputting the sex and/or weight and/or age of a sleeper.

### Example

A spray agent of an aqueous solution including 10% by mass of the GABA is sprayed by using each of a nebulizer of a ultrasonic system, a spray device of an ink jet system and a spray device of an electrostatic atomizing system, and a particle size distribution of the sprayed solution obtained in each spray is measured by using a QCM cascade impactor and a DMA (differential mobility analyzer). The measurement is performed in a position away from a spray port by 2 through 3 cm.

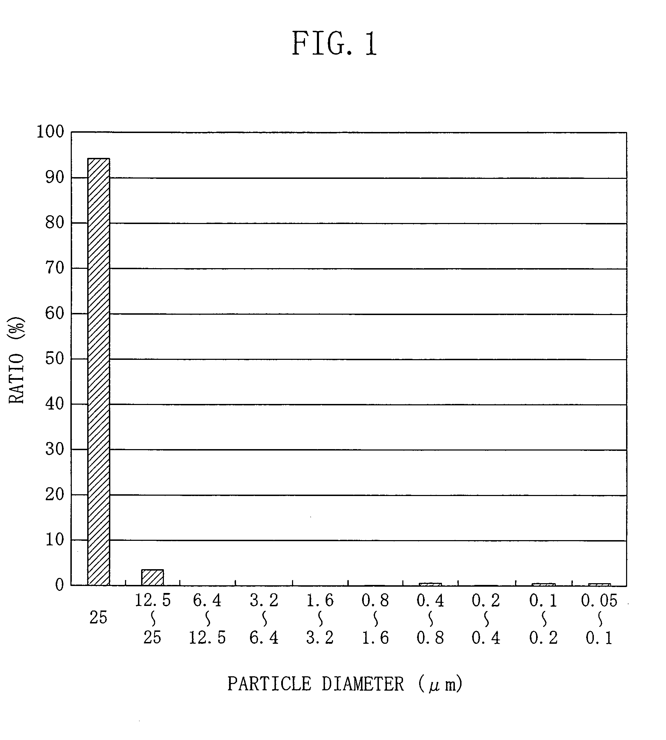

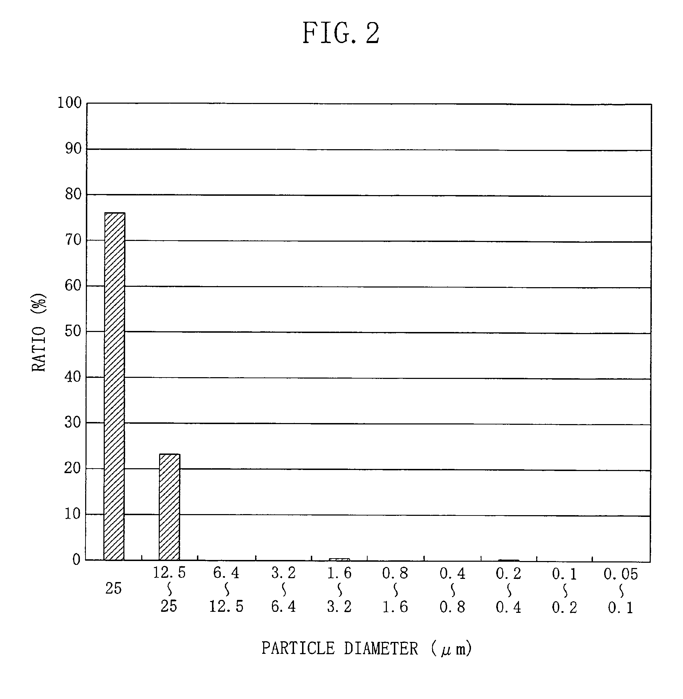

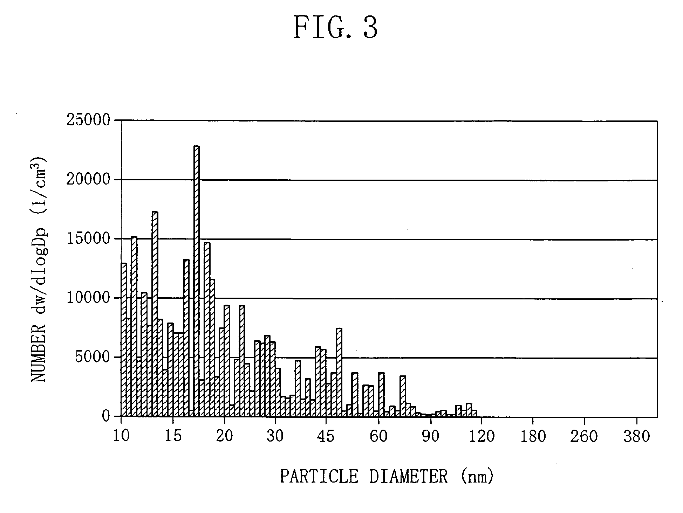

FIG. 1 shows the relationship between particle diameters of the sprayed solution and their ratios obtained by using the nebulizer of the ultrasonic system, FIG. 2 shows that obtained by using the spray device of the ink jet system and FIG. 3 shows that obtained by using the spray device of the electrostatic atomizing system. It is noted that FIGS. 1 and 2 show results measured by the QCM cascade impactor and FIG. 3 shows results measured by the DMA.

It is understood from these diagrams that the particle diameters of the sprayed solution are comparatively large and most of them are 25 µm in the spray by the nebulizer of the ultrasonic system and the spray device of the ink jet system while the particle diameters are smaller and most of them are 1 µm or less in the spray by the spray device of the electrostatic atomizing system. In the absorption through a lung, a particle diameter that can reach an air cell is 1 through 2 µm and the optimum particle diameter is said to be 0.1 µm. Therefore, among the spray devices used in this example, the spray device of the electrostatic atomizing system is suitably used.

### Industrial Applicability

As described so far, the present invention is useful for an atmosphere modifying method for a body space or a room space and a spray agent and a spray device used in the atmosphere modifying method.

## Claims

1. An atmosphere modifying method in which a solution including a solute of γ-aminobutyric acid dissolved in a solvent is sprayed in a space.

2. The atmosphere modifying method of Claim 1,
wherein said solution is sprayed in a particle diameter of 10 µm or less.

3. The atmosphere modifying method of Claim 1,
wherein a concentration of said γ-aminobutyric acid of 10 µg/m³ through 100 mg/m³ is attained in said space by the spray.

4. The atmosphere modifying method of Claim 1,
wherein the spray of said solution including said γ-aminobutyric acid is controlled in such a manner that a spray amount per unit time is larger at the start of the spray than in a steady spray time.

5. The atmosphere modifying method of Claim 1,
wherein the spray of said solution including said γ-aminobutyric acid is controlled in such a manner that a concentration of said γ-aminobutyric acid in said space is constant.

6. The atmosphere modifying method of Claim 1,
wherein the spray of said solution including said γ-aminobutyric acid is controlled to start when a given period of time elapses after detecting start of sleep.

7. A spray agent to be sprayed in a space comprising a solute of γ-aminobutyric acid dissolved in a solvent.

8. The spray agent of Claim 7,
wherein a concentration of said γ-aminobutyric acid is 0.0097 through 3.88 mol/1.

9. The spray agent of Claim 8,
wherein a concentration of said γ-aminobutyric acid is 0.097 through 1.94 mol/1.

10. A spray device constructed for spraying, in a space, a solution including a solute of γ-aminobutyric acid dissolved in a solvent.
